# FASCICULE DE BREVET EUROPEEN

(11) **EP 2 129 804 B1**
(45) Date de publication et mention de la délivrance du brevet: **18.03.2015**
(21) Numéro de dépôt: 08788066.2
(22) Date de dépôt: 28.03.2008
(51) Int. Cl.: C12Q 1/68

(54) **MÉTHODE DE DÉCONTAMINATION D'UNE SOLUTION DES ACIDES NUCLÉIQUES INDÉSIRABLES**
VERFAHREN ZUR DEKONTAMINIERUNG EINER LÖSUNG IN HINBLICK AUF UNERWÜNSCHTE NUKLEINSÄUREN
METHOD FOR DECONTAMINATING A SOLUTION WITH RESPECT TO UNWANTED NUCLEIC ACIDS

(30) Priorité: 30.03.2007 FR 0754165
(43) Date de publication de la demande: 09.12.2009
(73) Titulaire: BIOMERIEUX SA, 69280 Marcy L'Etoile (FR)
(72) Inventeur: PARANHOS-BACCALA, Glaucia, F-69003 Lyon (FR); LAURENT, Alain, F-38100 Grenoble (FR); LAAYOUN, Ali, F-38260 La Frette (FR)
(86) Numéro de dépôt international: PCT/FR2008/050540
(87) Numéro de publication internationale: WO 2008/132412

(56) Documents cités:
- WO-A-01/44507
- WO-A-93/20241
- WO-A-99/42832
- US-A1- 2005 191 682
- PITIE MARGUERITE ET AL: "Preparation of a spermine conjugate of the bis-phenanthroline ligand clip-phen and evaluation of the corresponding copper complex" BIOCONJUGATE CHEMISTRY, vol. 9, no. 5, septembre 1998 (1998-09), pages 604-611, XP002451073 ISSN: 1043-1802
- ASHKENAS JOHN ET AL: "Simple enzymatic means to neutralize DNA contamination in nucleic acid amplification" BIOTECHNIQUES, INFORMA LIFE SCIENCES PUBLISHING, WESTBOROUGH, MA, US, vol. 39, no. 1, juillet 2005 (2005-07), pages 69-73, XP001536574 ISSN: 0736-6205 cité dans la demande
- Marguerite Pitié ET AL: "DNA Cleavage by Copper Complexes of 2- and 3-Clip-Phen Derivatives", European Journal of Inorganic Chemistry, vol. 2003, no. 3, 1 February 2003 (2003-02-01), pages 528-540, XP055104637, ISSN: 1434-1948, DOI: 10.1002/ejic.200390075

## Description

La présente invention concerne une méthode de décontamination d'une solution des acides nucléiques indésirables. La présente invention concerne également l'utilisation d'une telle solution ainsi traitée.

L'essor de la biologie moléculaire a rendu possible la manipulation des acides nucléiques. Les méthodes d'amplification sont alors devenues un outil indispensable, en permettant d'obtenir à partir d'une très faible quantité d'acides nucléiques, une plus grande quantité dans un temps relativement court. Un inconvénient majeur de ces techniques réside dans l'amplification d'acides nucléiques indésirables, entraînant des résultats erronés, voire des faux positifs dans des tests cliniques. C'est ce que l'on appelle la contamination.

Cette contamination peut provenir de nombreuses sources : paillasses de laboratoire mal nettoyées, personnels, environnement, équipements et dispositifs de pipetage, échantillons non stériles.

Un certain nombre de recommandations visant à limiter ces contaminations a été mis en place. Il s'agit de méthodes préventives concernant par exemple la manipulation des échantillons (techniques de stérilisation notamment) ou encore les équipements de laboratoire (zones de travail physiquement délimitées, utilisation de hottes d'aspiration, gradient de pression entre l'extérieur et l'intérieur, afin que le flux permette toujours l'évacuation dans la direction souhaitée, etc.).

La contamination peut également provenir des amplicons issus d'amplifications précédentes ou d'une contamination croisée entre échantillons.

Une autre source non négligeable de contamination réside dans la matière première (enzymes, réactifs) utilisée dans les réactions d'amplification. Ainsi, Corless *et al*. exposent les méfaits de la contamination de la *taq* polymérase sur la sensibilité de la PCR en temps réel pour la détection de l'ARN 16S (J. Clin. Microbiol., 38(5), 1747-1752 (2000)).

Une première façon de remédier à la contamination des enzymes nécessaires à l'amplification, consiste en des méthodes de décontamination enzymatique.

Ainsi, le brevet US-A-5,418,149 décrit une méthode utilisant l'uracile-ADN-glycosylase qui permet de dégrader les acides nucléiques provenant de la cellules productrice de l'enzyme. Cette technique met en oeuvre la surexpression d'une protéine souhaitée, en l'occurrence une polymérase, dans des cellules d'*E*. *Coli* déficiantes en uracile-DNA glycosylase (UNG) et en deoxyuridine triphosphatase (dUTPase). Grâce à la déficience en UNG, la cellule n'élimine pas les bases deoxyuraciles incorporées. Par la déficience en dUTPase, la cellule augmente son pool de deoxyuridine. Ainsi le milieu de culture permet la production de la protéine souhaitée (polymérase) et l'incorporation de deoxyuridine dans les acides nucléiques. Les protéines synthétisées sont alors purifiées par des techniques standard de purification et par un traitement par l'uracile-ADN-glycosylase qui fragmente le résidu uracile des acides nucléiques résiduels. La glycosylase est ensuite inactivée par la chaleur de façon à éviter la destruction de l'ADN cible au cours d'une réaction d'amplification.

Cette méthode étant spécifique de la production de protéines recombinantes, son inconvénient majeur est dû à sa mise en oeuvre. De plus, la décontamination est limitée aux acides nucléiques contenant de l'uracile. Enfin, un autre inconvénient éventuel est que le traitement par la chaleur n'induit qu'une inactivation partielle de l'uracile-ADN-glycosylase. L'ADN cible mis en contact avec la protéine peut alors être éventuellement détruit.

Une autre méthode enzymatique décrite par Ashkenas et al. (Biotechniques; 2005 Jul.; 39(1):69-73), consiste à décontaminer une solution contenant l'ensemble des éléments nécessaires à une amplification. Il s'agit ici d'un cocktail d'enzymes de restriction utilisé dans le cadre d'une RT-PCR. Ces enzymes dégradent l'ADN double brin présent dans le milieu réactionnel d'amplification contenant l'ARN cible à amplifier. Elles sont ensuite inactivées par la chaleur lorsque la transcription inverse se produit. Une alternative à cette méthode pour une application PCR, donc en présence d'ADN cible double brin, est également décrite mais est limitée à l'utilisation d'un seul type d'enzyme de restriction (Type IIS RE). Toutefois, l'utilisation d'enzymes de restriction présente l'inconvénient de ne cliver que des acides nucléiques double brin qui en plus doivent présenter le site de reconnaissance. De ce fait, les contaminants sous forme simple brin et/ou ne présentant peu ou pas de tels sites ne sont alors pas éliminés.

D'autres méthodes enzymatiques de décontamination consistent, elles, à éliminer les acides nucléiques indésirables d'une solution avant la mise en contact avec les acides nucléiques cibles. La demande de brevet WO-A-99/07887 décrit l'utilisation d'une DNAse thermolabile permettant de dégrader les acides nucléiques double brin contenus dans le milieu réactionnel avant mise en contact avec les acides nucléiques cibles. L'enzyme est ensuite inactivée par la chaleur.

L'inconvénient majeur de cette technique est que cette enzyme ne permet pas la dégradation de contaminants sous forme d'ARN, d'ADN simple brin ou d'hétéroduplexes ARN/ADN. De plus, l'inactivation de cette enzyme par la chaleur dans le milieu réactionnel nécessite l'utilisation de polymérases thermostables.

L'art antérieur fait état également de méthodes non-enzymatiques.

Ainsi, Mohammadi et al. (J. Clin. Microbiol. 2003 Oct.; 41(10):4796-8) décrit une technique consistant en la filtration sur colonnes des réactifs d'extraction et éventuellement de la digestion par une enzyme de restriction, Sau3AI, des réactifs de la PCR avant amplification. L'inconvénient des techniques de filtration réside dans le fait que ces techniques ne peuvent pas être appliquées à des milieux complexes sans en changer la concentration ni les propriétés. De plus, cette étape supplémentaire de filtration peut elle-même être source de contamination.

La demande de brevet WO-A-94/12515 décrit une méthode de traitement par un composé photoréactif d'une solution contenant la *Taq* polymérase et potentiellement des acides nucléiques contaminants. Ce composé photoréactif, par exemple un dérivé furocoumarine, est activé par une exposition aux ultraviolets. Un inconvénient majeur de cette technique, au-delà de sa mise en oeuvre contraignante, est l'effet néfaste des radiations sur les protéines. De plus cette méthode reste peu efficace du fait de la dégradation aléatoire desdits acides nucléiques et peut générer des fragments encore amplifiables.

Une autre méthode non-enzymatique décrite par Chang et al., (RNA. 2005 May; 11(5):831-6) met en oeuvre un complexe de cobalt pour l'inhibition de la traduction. Ce complexe permet l'hydrolyse des liaisons phosphates diesters de l'ADN et de l'ARN.

Les inconvénients majeurs de cette technique sont la dégradation incomplète des acides nucléiques et la lenteur de la réaction de décontamination (24 heures).

Des complexes métalliques ont également été décrits dans la demande de brevet WO-A-2006/034009 afin de purifier des molécules biologiques contaminées par des acides nucléiques. Ainsi, un complexe de fragmentation constitué de deux molécules de phénanthroline associées à un atome de cuivre (bis (1,10 phénanthroline)/Cu) est utilisé pour purifier une solution contenant la *taq* polymérase. Cette solution est ensuite purifiée par des techniques standard de chromatographie afin d'enlever les acides nucléiques contaminants et/ou les molécules de fragmentation.

L'inconvénient majeur de cette méthode est la présence d'une étape de purification rendant difficile sa mise en oeuvre et augmentant le risque de nouvelles contaminations.

Le brevet EP-B-0.646.180 décrit un procédé permettant d'inactiver des séquences de nucléotides par des chelates métalliques. Un complexe de fragmentation tel que le bis (1,10 phénanthroline)/Cu est utilisé pour décontaminer les solutions, après l'étape d'amplification, de tous les acides nucléiques présents. Ceci permet d'éviter la contamination de nouveaux échantillons par des amplicons issus d'une réaction d'amplification antérieure. Ce document décrit également l'utilisation d'une molécule de fragmentation pour décontaminer des préparations de produits biologiques.

Dans le cas de la première utilisation, un inconvénient majeur de cette méthode est qu'elle ne permet pas la dégradation de tous les acides nucléiques contaminants mais seulement des amplicons issus d'une réaction d'amplification antérieure. Dans le deuxième cas, l'inconvénient majeur est la présence d'une étape de purification (ultrafiltration, précipitation, chromatographie) rendant difficile sa mise en oeuvre et augmentant le risque de nouvelles contaminations.

Compte tenu des inconvénients de l'art antérieur, les inventeurs proposent une nouvelle méthode simple et rapide de décontamination d'une solution des acides nucléiques indésirables.

La présente divulgation concerne une méthode de décontamination d'une solution de tout acide nucléique présent dans cette solution comprenant les étapes suivantes :
- soumettre, pendant une durée suffisante, la solution à l'action d'au moins un type de molécule ayant la propriété de dégrader les acides nucléiques par fragmentation, dite molécule de fragmentation, et ce jusqu'à la dégradation totale desdits acides nucléiques, dits acides nucléiques contaminants, et
- arrêter l'activité de la molécule de fragmentation.

L' invention concerne une méthode de décontamination d'une solution de tout acide nucléique présent dans cette solution comprenant les étapes suivantes :
- soumettre, pendant une durée suffisante, la solution à l'action d'au moins une molécule de fragmentation, formée par un complexe constitué de deux molécules de 1,10-phénanthroline associé à un atome métallique formant le complexe bis (1,10-phénanthroline)/métal, en présence de réducteur organique, jusqu'à la dégradation totale desdits acides nucléiques, et
- arrêter l'activité de la molécule de fragmentation par un ajout :
   - d'un excès de réducteur organique, ou
   - d'un agent complexant, tel que l'EDTA.

Un des avantages de la présente invention est que cette méthode de décontamination ne nécessite pas d'étape de purification pour éliminer les molécules de fragmentation.

Les acides nucléiques traités sont de l'ARN ou de l'ADN, se présentant sous forme simple ou double brin, ainsi que les hétéroduplexes ARN/ADN.

Cette méthode est particulièrement utile dans la décontamination de matières premières telles que par exemple les enzymes issues de sources naturelles comme les bactéries. Ces enzymes sont alors fortement contaminées par les acides nucléiques naturels issues de ces bactéries.

Un autre objet est une méthode de décontamination d'une solution, contenant des acides nucléiques contaminants et des acides nucléiques d'intérêt, et comprenant les mêmes étapes vues ci-dessus, à savoir :
- soumettre, pendant une durée suffisante, la solution à l'action d'au moins un type de molécule ayant la propriété de dégrader les acides nucléiques contaminants par fragmentation, dite molécule de fragmentation, et ce jusqu'à la dégradation desdits acides nucléiques contaminants, tout en conservant les acides nucléiques d'intérêt,
- arrêter l'activité de la molécule de fragmentation.

Les acides nucléiques contaminants peuvent être de l'ADN génomique humain et les acides nucléiques d'intérêt peuvent être des acides nucléiques viraux ou bactériens présents dans le mélange en très petite quantité. Ces acides nucléiques d'intérêt peuvent être protégés de l'action de la molécule de fragmentation. Ils peuvent, par exemple, être naturellement protégés par une enveloppe extérieure (capside, paroi bactérienne, etc.) alors que les acides nucléiques contaminants ne le sont pas.

L' invention a pour objet une méthode de décontamination d'une solution, contenant des acides nucléiques contaminants et des acides nucléiques d'intérêt comprenant les étapes suivantes :
- soumettre, pendant une durée suffisante, la solution à l'action d'au moins une molécule de fragmentation formée par un complexe constitué de deux molécules de 1,10-phénanthroline associé à un atome métallique formant le complexe bis (1,10-phénanthroline)/métal, en présence de réducteur organique, et ce jusqu'à la dégradation desdits acides nucléiques contaminants tout en conservant les acides nucléiques d'intérêt qui sont protégés de l'action de la molécule de fragmentation par une enveloppe extérieure, et
- arrêter l'activité de la molécule de fragmentation par un ajout :
   - d'un excès de réducteur organique, ou
   - d'un agent complexant, tel que l'EDTA.

Dans un mode avantageux de réalisation de l'invention, la méthode comprend, après l'arrêt de l'activité de la molécule de fragmentation, une étape supplémentaire qui consiste en une réaction d'amplification.

Préférentiellement, la méthode de décontamination selon l'invention est caractérisée en ce que la molécule de fragmentation utilisée a la propriété d'hydrolyser de manière aléatoire les liaisons phosphates diesters des acides nucléiques contaminants.

Selon un mode de réalisation, la molécule de fragmentation est un complexe constitué de deux molécules de 1,10-phénanthroline associé à un atome métallique formant le complexe bis (1,10-phénanthroline)/métal. Préférentiellement le métal est un métal de transition tel que le cuivre, le ruthénium, le nickel, le fer, le zinc, le rhodium, le cobalt, le manganèse.

De façon avantageuse, les deux noyaux phénanthroline de la molécule de fragmentation sont reliés l'un à l'autre par un bras de liaison pour former une molécule ClipPhen.
Les molécules ClipPhen ont été largement décrites dans la littérature. Les bras de liaison reliant les deux noyaux phénanthroline sont variés. De façon avantageuse, le bras de liaison entre les deux noyaux phénanthroline est constitué par une chaîne de trois atomes de carbone successifs, dont l'atome de carbone en position centrale est substitué et dont chaque atome de carbone terminal est relié à un noyau phénanthroline par un atome d'oxygène, tel que le serinol. Préférentiellement, l'atome de carbone en position centrale est substitué par NH₂ ou NH-CO-CH₃, et chaque atome de carbone terminal est relié à un noyau phénanthroline par un atome d'oxygène en position 2 ou 3 dudit noyau.

De nombreux autres bras sont possibles, comme par exemple un diamino-cyclohexane (Chemistry letters, Vol 33, n°6, p 684-985, Hayashi *et al*), ou encore un éthane diol, un propane diol, un pentane diol (Synlett 2001, n°10, 1629-1631, Boldron *et al*).

L'activité de fragmentation de la molécule ClipPhen associée à un métal est également connue de l'homme du métier (Chemical Review, 1993, 93, 2295-2316. D. Sigman *et al*. ; J. Chem. Soc. 1961, 2007-2019 James *et al*.). Cette molécule a fait l'objet de nombreux travaux dans le but d'améliorer ses propriétés d'hydrolyse. Elle présente l'avantage de dégrader tous les acides nucléiques, c'est-à-dire l'ADN et l'ARN sous forme simple ou double brin ainsi que les hétéroduplexes ARN/ADN. Ainsi la 2-ClipPhen/métal (1,3-bis(1,10-phenanthrolin-2-yloxy)propan-2-amine)/métal et la 3-ClipPhen/métal (1,3-bis(1,10-phenanthrolin-3-yloxy)propan-2-amine)/métal ont montré une activité nucléasique largement augmentée par rapport au complexe bis (1,10-phénanthroline)/métal (Inorganic Chemistry 1998, 3486-3489, Pitié *et al*. ; Chem. Commun. 1998, 2597-2598, Pitié *et al*. ; Eur. J. Inorg. Chem. 2003, 528-540, Pitié *et al*. *et* Bioconjugate Chemistry 2000, 11, 892-900 Pitié M. *et al*. ). De même une molécule ClipPhen complexée au cuivre a la propriété d'hydrolyser très efficacement et de façon aléatoire les liaisons phosphates diesters de l'ADN ou de l'ARN. Elle peut se complexer avec un atome de cuivre pour donner des complexes d'état d'oxydation I ou II tels que montrés dans la publication J. Chem. Soc. 1961, 2007-2019 James *et al*. Les molécules 2-ClipPhen-Cu et 3-ClipPhen-Cu sont représentées ci-dessous :

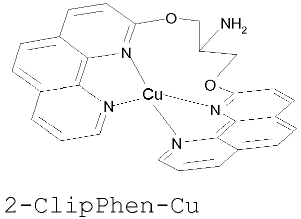

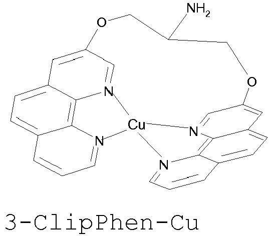

Ainsi, la présente divulgation a également pour objet une méthode de décontamination d'une solution de tout acide nucléique présent dans cette solution consistant à soumettre, pendant une durée suffisante, la solution à l'action de la molécule ClipPhen/métal, et ce jusqu'à la dégradation totale des acides nucléiques contaminants.

Un autre objet de la présente divulgation consiste en une méthode de décontamination d'une solution, contenant des acides nucléiques contaminants et des acides nucléiques d'intérêt consistant à soumettre, pendant une durée suffisante, la solution à l'action de la molécule ClipPhen/métal, et ce jusqu'à la dégradation desdits acides nucléiques contaminants, tout en conservant les acides nucléiques d'intérêt.

Selon un mode de réalisation de la présente invention, l'activité de fragmentation de la molécule de fragmentation est arrêtée par un ajout d'un excès de réducteur organique. Lorsque le réducteur organique est constitué par un thiol, le rapport entre la molécule de fragmentation et le réducteur organique arrêtant la réaction est supérieur à 1 pour 100, et préférentiellement supérieur à 1 pour 1000.

Un avantage de ce mode de réalisation est que les molécules de fragmentation sont inactivées *in situ.* Ces molécules n'ont alors pas besoin d'être retirées de la solution. Cette dernière pourra être utilisée, telle quelle, dans diverses applications comme par exemple une amplification.

Ainsi, en fonction de la concentration du réducteur organique présent, les molécules de fragmentation peuvent être activées ou désactivées.

Selon un autre mode de réalisation de la présente invention, l'activité de fragmentation de la molécule de fragmentation est arrêtée par un ajout d'un agent complexant (AC) comme par exemple l'EDTA, ou tout autre complexant possédant plus d'affinité pour le métal que la ClipPhen et permettant un déplacement de l'équilibre :

ClipPhen-métal + AC → ClipPhen + métal-AC

Dans un mode de réalisation particulier de la présente invention la molécule de fragmentation est un complexe cuivreux de type I associé à du péroxyde d'hydrogène H₂O₂ et, éventuellement, à un autre réducteur préférentiellement organique. De préférence, la molécule de fragmentation est une molécule ClipPhen/CuI.

Dans le cas d'un complexe cuivreux de type I, l'activité nucléasique procède en deux temps :
a) le complexe cuivreux chargé positivement se complexe à l'acide nucléique,
b) l'acide nucléique est oxydé par formation d'un intermédiaire Cu-oxo ou Cu-OH avec H₂O₂ qui conduit, après une suite de réactions, à la coupure de l'acide nucléique.

Selon un autre mode de réalisation, la molécule est un complexe cuivrique de type II associé à un autre réducteur préférentiellement organique. Dans ce cas, le péroxyde d'hydrogène est généré *in-situ* sous l'action de l'air et du réducteur. De préférence alors, la molécule de fragmentation est une molécule ClipPhen/CuII.

Lorsque l'autre réducteur organique est un acide carboxylique ou un dérivé d'un tel acide, le rapport entre la molécule de fragmentation et le réducteur organique est compris entre 1 pour 1 et 1 pour 100.

Selon les modes de réalisation précédents, le réducteur organique est constitué par :
- un thiol, tel que le di-thiothréitol (DTT), un thio-acide, tel que l'acide mercapto-propionique ou
- un acide carboxylique ou
- un dérivé d'un acide carboxylique tel que l'ascorbate ou
- une phosphine comme la tri carboxy ethyl phosphine (TCEP).

Dans un mode de réalisation particulier, la molécule de fragmentation et/ou le réducteur est immobilisé sur un support solide. Ce support solide peut être une particule de latex (éventuellement magnétique), de verre (CPG), de silice, de polystyrène, d'agarose, de sépharose, de nylon, etc. Il peut s'agir également d'un filtre, d'une membrane ou d'une bandelette ou d'un film.

L'activité de fragmentation de la molécule de fragmentation peut également être arrêtée lorsque le support solide sur lequel est fixé la molécule de fragmentation et/ou le réducteur est retiré de la solution.
La molécule de fragmentation peut être fixée par liaison covalente ou par adsorption sur les supports considérés. De nombreuses références concernant les réactifs supportés existent, on pourra notamment se référer à Laurent et al., Tetrahedron Letters 45, 8883-8887, 2004.

Un autre avantage de la présente invention par rapport à l'art antérieur est la rapidité de la réaction subie par les acides nucléiques. La durée du traitement est comprise entre 5 et 60 minutes préférentiellement entre 10 et 30 minutes pour des concentrations en molécules ClipPhen/métal comprises entre 1 nM et 100 µM.

Dans un mode préféré de l'invention, la solution contient tous les constituants nécessaires à une réaction d'amplification à l'exception des acides nucléiques soit les cibles, les amorces d'amplification et les sondes de détection.

Un autre objet de la présente invention est l'utilisation d'une solution traitée selon la présente invention, la solution étant mélangée avec un échantillon biologique contenant des acides nucléiques cibles, que l'on souhaite amplifier, mais également les amorces d'amplification et les sondes de détection spécifiques des acides nucléiques cibles en présence du réducteur organique, créant ainsi un excès de réducteur organique ce qui arrête l'action des molécules de fragmentation.

Le réducteur organique qui arrête l'action des molécules de fragmentation peut être identique ou différent de l'autre réducteur organique ajouté préalablement pour activer la réaction nucléasique de la molécule de fragmentation.

### Définitions

Le terme « acide nucléique » signifie un enchaînement d'au moins deux désoxyribonucléotides ou ribonucléotides.

L'acide nucléique peut être naturel ou synthétique, un oligonucléotide, un polynucléotide, un fragment d'acide nucléique, un ADN génomique, un ARN ribosomique, un ARN messager, un ARN de transfert, un acide nucléique obtenu par une technique d'amplification enzymatique, telle que :
- PCR (Polymerase Chain Reaction), décrite dans les brevets US-A-4,683,195, US-A-4,683,202 et US-A-4,800,159, et sa dérivée RT-PCR (Reverse Transcription PCR), notamment dans un format en une étape, tel que décrit dans le brevet EP-B-0.569.272,
- LCR (Ligase Chain Reaction), exposée par exemple dans la demande de brevet EP-A-0.201.184,
- RCR (Repair Chain Reaction), décrite dans la demande de brevet WO-A-90/01069,
- 3SR (Self Sustained Sequence Replication) avec la demande de brevet WO-A-90/06995,
- NASBA (Nucleic Acid Sequence-Based Amplification) avec la demande de brevet WO-A-91/02818, et
- TMA (Transcription Mediated Amplification) avec le brevet US-A-5,399,491.

On parle alors d'amplicons pour désigner les acides nucléiques générés par une technique d'amplification enzymatique.

Par « tout acide nucléique » on entend les séquences d'ARN, d'ADN, sous la forme de simple ou double brin ainsi que les hétéroduplexes ARN/ADN.

On entend par « arrêter l'activité de la molécule de fragmentation » le fait:
- d'ajouter d'un excès de réducteur organique ou
- d'ajouter d'un agent complexant.

Le terme « dégradation » signifie la dégradation ou la fragmentation d'au moins 90% des acides nucléiques contaminants. De préférence la dégradation est dite « totale » ce qui signifie que la dégradation d'ADN ou d'ARN, préférentiellement par une hydrolyse des liaisons phosphate diesters, entraîne la formation de séquences d'acides nucléiques « non amplifiables ».

On entend par des acides nucléiques « non amplifiables » des acides nucléiques qui, en présence d'amorces dont la taille est préférentiellement au moins de 10 nucléotides, ne peuvent pas être amplifiés. La taille de ces acides nucléiques « non amplifiables » est donc inférieure à 20 nucléotides, préférentiellement inférieure à 10 nucléotides.

Le terme « solution » signifie une solution aqueuse homogène ou hétérogène. Il peut s'agir d'une solution complexe contenant des enzymes, des molécules organiques (nucléosides triphosphates, sucres, saccharides, polysaccharides, petites molécules organiques, etc.), des molécules inorganiques (sels, etc) et cela en mélange ou non. La solution peut également contenir un support solide telles que des particules qui peuvent être en latex éventuellement magnétique, en verre (CPG), en silice, en polystyrène, en agarose, en sépharose, en nylon, etc. Il peut s'agir également d'un filtre, d'un film, d'une membrane ou d'une bandelette.

La solution peut également être constituée par une solution en contact avec une surface solide tel un tube de plastique (type Eppendorf), permettant ainsi la décontamination de cette surface.

Les figures et exemples ci-joints représentent des modes particuliers de réalisation et ne peuvent pas être considérés comme limitant la portée de la présente invention.

La portée de l'invention est limitée par les revendications 1 à 21.

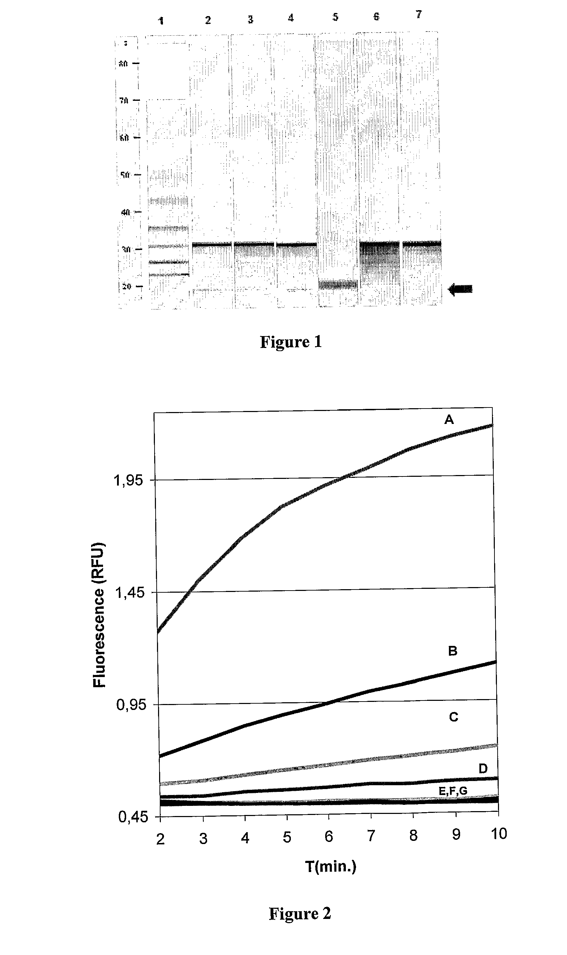

Figure 1 : Etude au Bioanalyser (Agilent, Kit RNA 6000 Pico, USA) de la dégradation d'un transcrit de 1080 bases (*Salmonella* type sauvage) soumis à l'action du mélange ClipPhen-Cu/Ascorbate pendant 1 heure à 37°C. La bande sombre située à 31 secondes dans les colonnes 2, 3, 4, 6 et 7 correspond au transcrit de 1080 bases.
- Colonne 1 : Echelle de taille en nucléotides. Injection d'une gamme d'ARN de taille croissante, permettant la quantification et le calcul de la taille des acides nucléiques analysés, en fonction de leur moment de passage devant le détecteur du bioanalyser. L'unité de mesure sur l'axe vertical est la seconde. La conversion entre le temps de passage et la masse des acides nucléiques est faite par le logiciel du bioAnalyser.
- Colonne 2 : Transcrit 5 nM (nanoMolaire) en présence de 5 µM (microMolaire) ClipPhen-Cu.
- Colonne 3 : Transcrit 5 nM en présence de 500 nM ClipPhen-Cu.
- Colonne 4 : Transcrit 5 nM en présence de 50 nM ClipPhen-Cu.
- Colonne 5 : idem colonne 2 avec rajoût d'ascorbate 500 µM.
- Colonne 6 . idem colonne 3 avec rajoût d'ascorbate 50 µM.
- Colonne 7 : idem colonne 4 avec rajoût d'ascorbate 5 µM.

Figure 2 : Inhibition de la fragmentation des acides nucléiques par augmentation de la concentration en DTT (0 à 16 mM).
La courbe A correspond à 0 mM de DTT.
La courbe B correspond à 1 mM de DTT.
La courbe C correspond à 3 mM de DTT.
La courbe D correspond à 5 mM de DTT.
La courbe E correspond à 10 mM de DTT.
La courbe F correspond à 16 mM de DTT.
La courbe G correspond au témoin : 0mM de DTT et 0 mM d'ascorbate.

Figure 3 : Vitesse de fragmentation de l'oligonucléotide fluorigène modèle en fonction de la concentration en DTT dans le mélange (en unité de fluorescence relative (RFU) par minute).

Figure 4 : Effet du mélange ClipPhen-Cu/ascorbate sur une solution aqueuse contenant des acides nucléiques (100 équivalent cellule / µl d'ARN totaux de *B. Cereus*).
La courbe A correspond au témoin d'amplification n'ayant subit aucun traitement (contrôle +).
La courbe B correspond au mélange ClipPhen-Cu 50 µM/ascorbate 500 µM dont l'activité est immédiatement inhibée par ajoût de 17 mM de DTT et qui est incubé pendant 30 minutes.
La courbe C correspond au mélange ClipPhen-Cu 50 µM/ascorbate 500 µM incubé 30 minutes avec arrêt de la fragmentation avec du DTT au bout de ce temps.

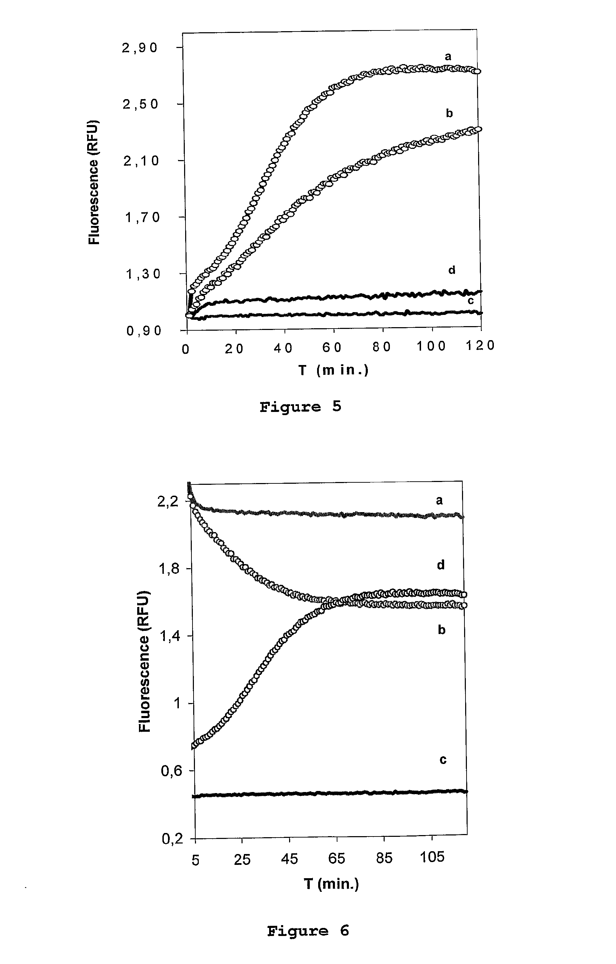

Figure 5 : Comparaison de la fragmentation de l'ADN ou de l'ARN simple brin par la ClipPhen-Cu (avec ou sans ascorbate). Courbes normalisées à 1.
Courbe a : Oligonucléotide fluorigène (ADN) n° 16025 soumis à l'action du mélange ClipPhen-Cu/ascorbate.
Courbe b : Oligonucléotide fluorigène (ARN) n° 16027 soumis à l'action du mélange ClipPhen-Cu/ascorbate.
Courbe c : Témoin négatif de l'expérience a : Oligonucléotide fluorigène (ADN) n° 16025 soumis à l'action du mélange ClipPhen-Cu seulement.
Courbe d : Témoin négatif de l'expérience b : Oligonucléotide fluorigène (ARN) n° 16027 soumis à l'action du mélange ClipPhen-Cu seulement.

Figure 6 : Comparaison de la fragmentation d'ADN simple ou double brin par le mélange ClipPhen-Cu (avec ou sans ascorbate). Les courbes représentent une vitesse de fragmentation correspondant à une augmentation de la fluorescence par minute (RFU/min).
Courbe a : Duplex oligonucléotide fluorigène (ADN) n° 16025 /cible complémentaire ADN (n° 16026) soumis à l'action du mélange ClipPhen-Cu (témoin négatif).
Courbe b : Duplex oligonucléotide fluorigène (ADN) n° 16025 /cible complémentaire ADN (n° 16026) soumis à l'action du mélange ClipPhen-Cu/ascorbate.
Courbe c : Oligonucléotide fluorigène (ADN) n° 16025 soumis à l'action du mélange ClipPhen-Cu.(témoin négatif).
Courbe d : Oligonucléotide fluorigène (ADN) n° 16025 soumis à l'action du mélange ClipPhen-Cu/ascorbate.

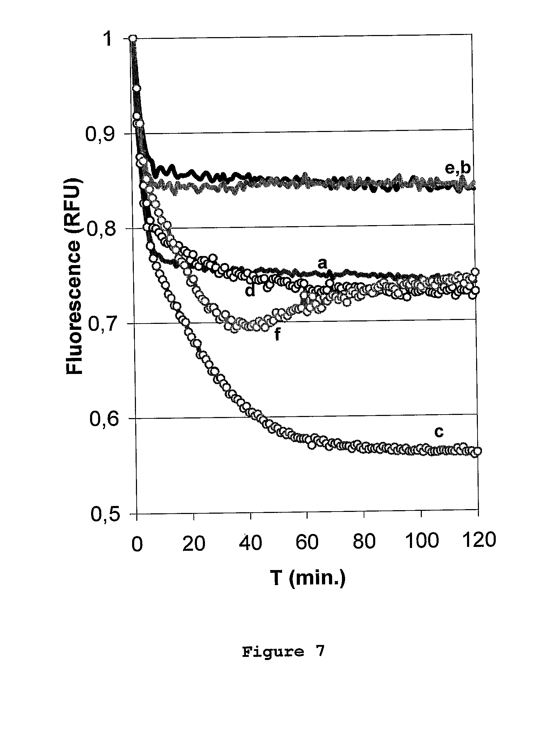

Figure 7 : Comparaison de la fragmentation de duplex ADN ou ARN ou d'un héteroduplex ARN/ADN par la ClipPhen-Cu (avec ou sans ascorbate). Courbes normalisées à 1.
Courbe a : Duplex oligonucléotide fluorigène (ADN) n° 16025 /cible complémentaire ADN (n° 16026) soumis à l'action du mélange ClipPhen-Cu (témoin négatif).
Courbe b : Duplex oligonucléotide fluorigène (ARN) n° 16027 /cible complémentaire ARN (n° 16028) soumis à l'action du mélange ClipPhen-Cu (témoin négatif).
courbe c : Duplex oligonucléotide fluorigène (ADN) n° 16025 /cible complémentaire ADN (n° 16026) soumis à l'action du mélange ClipPhen-Cu/ascorbate.
courbe d : Duplex oligonucléotide fluorigène (ARN) n° 16027 /cible complémentaire ARN (n° 16028) soumis à l'action du mélange ClipPhen-Cu/ascorbate.
Courbe e : Hétéro duplex oligonucléotide fluorigène (ARN) n° 16027 /cible complémentaire ADN (n° 16026) soumis à l'action du mélange ClipPhen-Cu (témoin négatif).
Courbe f : Hétéro duplex oligonucléotide fluorigène (ARN) n° 16027 /cible complémentaire ADN (n° 16026) soumis à l'action du mélange ClipPhen-Cu/ascorbate.

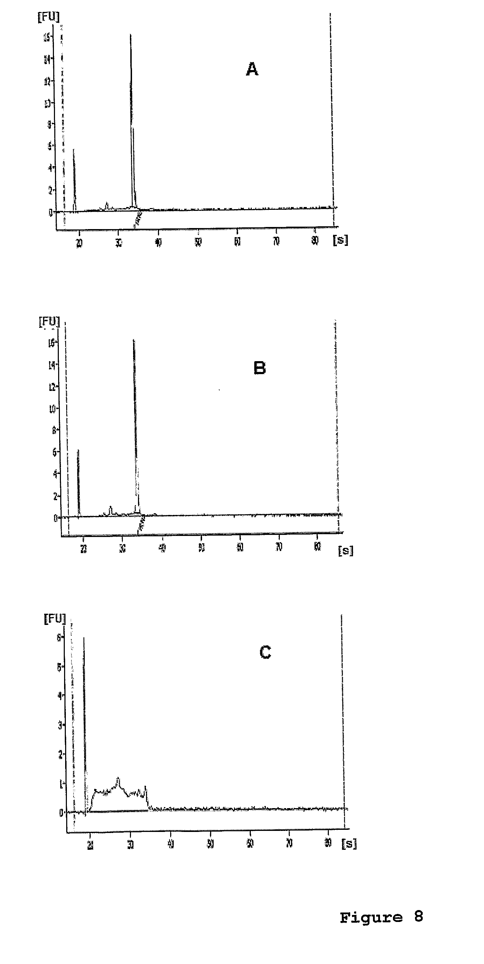

Figure 8: Observation au Bioanalyseur (Agilent) du clivage de l'ARN 16s (13ng/µL) par la ClipPhen-Cu immobilisée sur une bille TentaGel en présence d'ascorbate 5mM.
Electrophoregramme A : Cible ARN avant dégradation dans l'eau ;
Electrophoregramme B : Cible ARN avant dégradation en présence d'ascorbate ;
Electrophoregramme C : Cible ARN dégradée en présence d'ascorbate et de la ClipPhen-Cu immobilisée sur résine Tentagel (1 bille de Tentagel, 30 min) ;

### Exemple 1 Hydrolyse d'un transcrit par la molécule ClipPhen-Cu et effet initiateur de l'ascorbate.

### But :

Cet exemple démontre l'action de fragmentation du mélange ClipPhen-Cu/ascorbate sur un acide nucléique modèle (Transcrit ARN Salmonelle : 1080 bases). Il démontre également l'effet de l'ascorbate sur l'initiation de la réaction de fragmentation. Le transcrit est soumis à l'action de différentes concentrations du mélange ClipPhen-Cu/ascorbate. Le témoin est soumis également à l'action de la ClipPhen-Cu mais en absence d'ascorbate.

Les fragments sont ensuite analysés à l'aide du Bionalyser d'Agilent (figure 1).

### Mode opératoire :

Une solution de transcrit (Salmonella sauvage 1080 base) est constituée à partir de 2 µl de transcrit à 1 µM dans l'eau et de 18 µl de tampon phosphate 80 mM (milliMolaire) pH 7,2, MgCl₂ 20 mM, NaCl 100 mM. Cette solution est portée à 70°C pendant 2 minutes puis mise dans la glace.

On prélève alors 5µl de cette solution à laquelle on rajoute 1 µl de ClipPhen-Cu (500, 50 et 5µM) et 4 µl du tampon utilisé précédement. On obtient la solution 2 que l'on laisse 1h à 37°C.

On prend alors 5 µl de la solution 2 et on y ajoute 42,5 µl de tampon et 2,5 µl d'une solution d'ascorbate fraîchement préparée (100 eq par rapport à la [ClipPhen-Cu]) ou d'eau dans le cas des témoins.

Ainsi on se retrouve avec un série de tubes comportant respectivement 5 nM de transcrit, (5 µM, 500 nM et 50 nM) de ClipPhen-Cu et (500, 50 et 5 µM) d'ascorbate ou d'eau pour les témoins.

On injecte immédiatement 1 µl des 6 solutions sur la puce RNA 6000 Pico (Kit commercial référencé 5067-1512, Agilent, USA), utilisé sur le système BioAnalyzer d'Agilent ,USA) pour observer les produits de fragmentation.

### Résultats et conclusions :

On observe l'effet de la fragmentation sur le transcrit colonnes 5 à 7 par l'apparition de bandes de différentes tailles et inférieures à 20 nucléotides (flèche sur la droite au niveau de 20 secondes). Ainsi le transcrit ayant subit le traitement par le mélange ClipPhen-Cu/ascorbate est partiellement dégradé ou totalement dégradé en fonction de la concentration en ClipPhen-Cu. Entre 5 µM et 0,5 µM, on montre la disparition de la bande initiale. Le temps de réaction permettant d'obtenir une dégradation totale de tous les amplicons est de l'ordre de 30-60 minutes avec 5 nM de transcrit, 5 µM de ClipPhen-Cu et 500 µM d'ascorbate. La quantité de ClipPhen-Cu pour la dégradation totale du transcrit est de l'ordre du micromolaire (colonne 5).

Par contre la même expérience effectuée sans ascorbate (colonne 2) montre bien une bande intacte.

On démontre ainsi que le mélange ClipPhen-Cu n'a aucune activité tant que l'ascorbate n'a pas été ajouté (excès de l'ordre de 1-100 équivalents).

### Exemple 2 Inhibition de la molécule ClipPhen-Cu par un réducteur.

### But :

Cet exemple vise à montrer que la dégradation des acides nucléiques par le mélange ClipPhen-Cu/ascorbate peut être totalement inhibée par l'ajout d'un excès de réducteur comme le DTT.

Un oligonucléotide fluorigène modèle (Seq 16025 synthétisée par Eurogentec (Liège, Belgique) : 5' (6-FAM) TGT AAT GAT GAG GGT GTC ACT GCG GTT (TAMRA) ) est soumis à l'action du mélange ClipPhen-Cu /ascorbate. En se fragmentant, il laisse apparaître de la fluorescence à 520 nm car le fluorophore FAM se retrouve éloigné physiquement de l'extincteur de fluorescence TAMRA. L'apparition de fluorescence est mesurée au cours du temps à l'aide d'un fluorimètre (NucliSens EasyQ Analyser, Nasba Diagnostic, bioMérieux, Boxtel (NL)). L'expérience est effectuée avec des concentrations croissantes de DTT pour une concentration fixe de ClipPhen-Cu/ascorbate afin de mesurer l'inhibition apportée par le DTT par atténuation de la fluorescence émise.

### Mode opératoire :

Une série de tubes contenant les éléments ci-dessous est mise à incuber à 37°C pendant 5 minutes :
- l'oligonucléotide fluorigène 16025 à 10 nM (1 µl à 200 nM),
- le DTT (1 µl à une concentration telle pour être en suite de 1 à 16 mM dans le volume final), et
- 17 µl de tampon phosphate 80 mM pH 7,2, MgCl₂ 20 mM, NaCl 100 mM.

On rajoute alors 1 µl d'un mélange ClipPhen-Cu/Ascorbate préparé de la façon suivante : 200 µM ClipPhen et 200 µM CuCl₂ dans DMF/eau 50/50 et ascorbate 20 mM dans l'eau préparé extemporanément (à partir de solutions concentrées à 10 mM de ClipPhen-Cu et à 1 M d'ascorbate).

Au final, les 20 µl de solution contiennent : 10 nM d'oligonucléotide fluorigène, 10 µM clip-phen + Cu²⁺, 1 mM ascorbate et 0/1/3/5/10/16 mM de DTT.

Un témoin est également réalisé sans ascorbate et sans ClipPhen-Cu. On commence alors la mesure de la fluorescence émise pendant 60 minutes (Figure 2). Les conditions sont les suivantes : filtres exc/em (485/518 nm), intervalle de temps (1 min), temps d'intégration (100 ms).

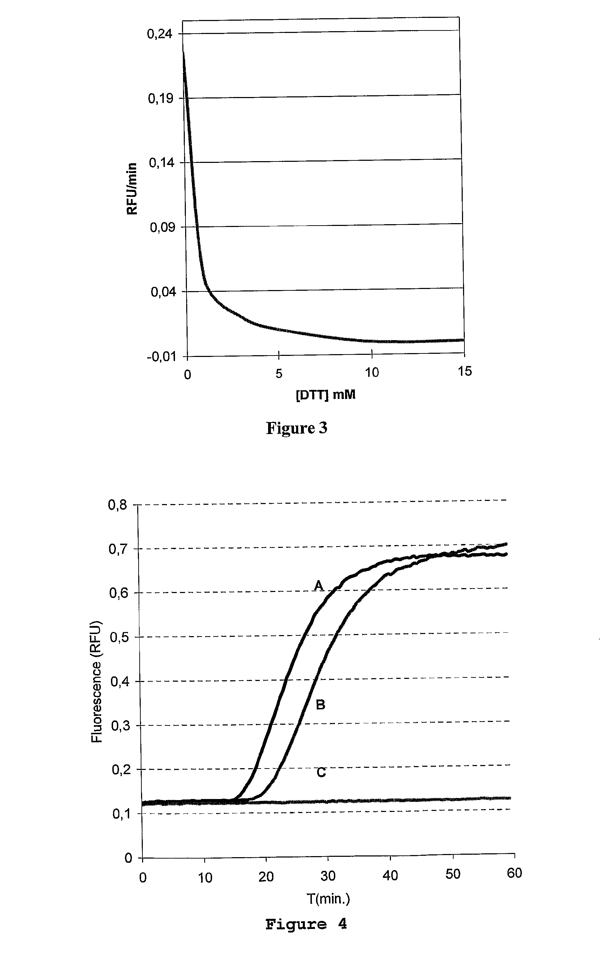

A partir des courbes obtenues, on mesure la vitesse de la réaction de fragmentation sur les 2 premières minutes de la réaction et on trace le graphe, Figure 3, représentant la vitesse de fragmentation en fonction de la concentration en DTT.

### Résultats et conclusions :

On constate que pour des concentrations croissantes en DTT, la fluorescence n'apparaît plus, et cela à partir de 10 mM (Figures 2 et 3). Ce qui signifie que l'activité du mélange ClipPhen-Cu/Ascorbate est totalement arrêtée par excès de réducteur. Cette expérience démontre que l'ajoût de 10-16 mM de DTT au mélange acides nucléiques/CliPhen-Cu/Ascorbate permet d'arrêter la réaction et de rendre ce mélange compatible avec une future utilisation dans une réaction d'amplification.

### Exemple 3 Effet décontaminant de la CliPhen dans une solution aqueuse.

### But :

Décontamination d'une solution aqueuse contaminée par un mélange d'ARN totaux de *B. Cereus* à hauteur de 200 équivalent cellule/µl soumise à l'action du mélange ClipPhen-Cu/ascorbate (50 µM / 500 µM) pendant 30 minutes. L'activité de la ClipPhen-Cu est ensuite totalement arrêtée par ajoût de DTT à hauteur de 10 mM. On effectue alors sur ce mélange une réaction d'amplification NASBA (Nuclisens Basic Kit V2 de bioMérieux, référencé 60079136, Boxtel (NL)) pour évaluer l'efficacité de la fragmentation et montrer l'absence d'acides nucléiques amplifiables. Il n'est pas nécessaire d'effectuer sur ce mélange une purification préalablement à l'amplification. Un témoin est effectué dans lequel l'activité de la ClipPhen-Cu est inhibée dès t0 afin de mesurer l'éventuel effet inhibiteur de cette dernière. Pour cela on ajoute le DTT en conditions suffisantes (10 mM) avant d'ajouter la cible ARN et on maintient une incubation de 30 minutes.

### Mode opératoire :

### Décontamination par le mélange ClipPhen-Cu/ascorbate d'une solution aqueuse enrichie en ARN totaux.

A une solution d'ARN totaux d'ARN de *B. Cereus* (5 µl à 2000 équivalent Cellule/µl dans l'eau) sont ajoutés 10 µl de ClipPhen-Cu (250 µM dans eau/DMF 50/50) 20 µl d'eau et 10 µl d'ascorbate à 2,5 mM dans l'eau.

On incube cette solution à décontaminer pendant 30 minutes à température ambiante.

### Arrêt de l'activité de fragmentation du mélange ClipPhen-Cu/ascorbate.

On rajoute 5 µl de DTT à 173,7 mM (concentration finale DTT : 17,3 mM) à la solution précédente pour arrêter la réaction d'hydrolyse et désactiver la ClipPhen. On obtient la solution A.

### Témoin pour montrer que le mélange ClipPhen-Cu/Ascorbate/DTT n'a pas d'activité de fragmentation et n'inhibe pas la NASBA si l'activité de fragmentation est immédiatement arrêtée par l'ajout de DTT.

Parallèlement est réalisée une expérience témoin dans laquelle une solution d'ARN totaux d'ARN de *B. Cereus* (5 µl à 2000 équivalent Cellule/µl dans l'eau) est incubée en présence de 5 µl de DTT à 173,7 mM, 10 µl de ClipPhen-Cu (250 µM dans eau/DMF 50/50, 20 µl d'eau et 10 µl d'ascorbate à 2,5 mM dans l'eau). On incube cette solution dans laquelle le mélange ClipPhen-Cu/ascorbate est immédiatement inactivé pendant 30 minutes à température ambiante.

Cette expérience permet de mesurer l'éventuelle inhibition de la réaction d'amplification par le mélange ClipPhen-Cu/ascorbate/DTT. Il s'agit de la solution B.

### Amplification des acides nucléiques restant dans la solution A après décontamination et toujours présent dans la solution B.

On prélève alors 5 µl des solutions A ou B auxquelles on rajoute, 10 µl du mélange « Reactif Mix : REAG » (du NucliSens EasyQ Basic Kit bioMérieux (référence commerciale 285006, Boxtel (NL)). Ce mélange contient les réactifs nécessaires à l'amplification , les deux amorces et la sonde nécessaire à la détection de l'amplification. Cette solution contient en outre suffisamment de DTT (6 mM) pour empêcher une réactivation de la ClipPhen. La concentration finale du mélange est donc de 10 mM en DTT.Les solutions sont mises à incuber 2 minutes à 65°C puis 2 minutes à 41°C.

On rajoute alors aux solutions précédentes 5 µl du mélange « Enzyme Mix : ENZ» du Basic Kit NucliSens bioMérieux afin de commencer l'amplification. Le milieu réactionnel est maintenu 1h30 à 41°C dans le fluorimètre « NucliSens EasyQ » de bioMérieux (NucliSens EasyQ Analyser, Nasba Diagnostic, bioMérieux, Boxtel (NL)). Les mesures de fluorescence sont enregistrées et les courbes tracées (Figure 4).

### Résultats et conclusions :

On observe un léger décalage de la courbe B qui correspond au témoin ayant subit l'action du mélange ClipPhen-Cu/ascorbate immédiatement inhibée par ajoût de la solution de DTT. Ce décalage de l'amplification ne change en rien la sensibilité du test puisque les pentes au démarrage de l'amplification sont de même amplitude. Il est dû à une augmentation de la concentration en DTT par rapport au témoin positif (Courbe A). On montre dans ce cas que le mélange ClipPhen-Cu/ascorbate/DTT n'a pas d'effet sur la NASBA et que les enzymes de cette réaction d'amplification ne sont pas affectées par ce mélange de composés. De plus, on montre également que le DTT permet l'inhibition de l'activité de fragmentation de la ClipPhen-Cu puisqu'aucune activité de dégradation n'est notée quand on rajoute suffisamment de DTT.

La courbe C montre que si l'activité du mélange ClipPhen-Cu/ascorbate est maintenue pendant 30 minutes puis stoppée par ajoût de DTT (17 mM), la fragmentation est telle que l'on ne décèle plus de courbe d'amplification.
On démontre ici que l'on peut utiliser un mélange complexe (ClipPhen-Cu/ascorbate) pour dégrader des acides nucléiques, arrêter cette activité par ajoût d'un excès de DTT et utiliser cette solution (sans purification) pour une amplification enzymatique ultérieure. Cette technique permet donc avec une grande facilité de s'affranchir de problèmes de contamination par des acides nucléiques.

### Exemple 4 Fragmentation par la ClipPhen-Cu d'acides nucléiques autres que l'ARN ; application à l'ADN simple brin, l'ADN double brin à l'ARN double brin et un hétéroduplex ARN/ADN.

### But :

Cet exemple démontre que le mélange ClipPhen-Cu/ascorbate peut aussi bien dégrader de l'ARN ou de l'ADN simple brin que les duplex ou hétéroduplex correspondants.

### Mode opératoire :

On prépare les solutions indiquées dans le tableau ci dessous à partir d'oligonucléotides fluorigènes (les sondes) et de cibles complémentaires commandés chez Eurogentec (Tableau 1).

**Tableau 1: Séquences utilisées dans l'exemple 4.**

| Commentaires | Ref ODN | Séquence | Modification 5' | Modification 3' |
|---|---|---|---|---|
| ADN sonde fluorigène | 16025 | TGT AAT GAT GAG GGT GTC ACT GCG GTT | 6-FAM | TAMRA |
| ARN sonde fluorigène | 16027 | UGU AAU GAU GAG GGU GUC ACU GCG GUU | 6-FAM | TAMRA |
| ADN cible complémentaire | 16026 | AAC CGC AGT GAC ACC CTC ATC ATT ACA | - | - |
| ARN cible complémentaire | 16028 | AAC CGC AGU GAC ACC CUC AUC AUU ACA | - | - |

Les solutions mères d'oligonucléotides fluorigènes sont à 200 nM dans l'eau que l'on dilue à 10 nM (1 µl) dans 18 µl de tampon phosphate 80 mM pH 7,2, MgCl₂ 20 mM, NaCl 100 mM. Selon les cas, la cible complémentaire est ajoutée à la même concentration. Les solutions sont alors dénaturées et laissées à 37°C pendant une heure afin de permettre l'hybridation des deux brins. On rajoute alors une solution de Cliphen-Cu (1 µl à 20 µM dans eau/DMF, [ClipPhen-Cu finale] = 1 µM) puis une solution d'ascorbate (1 µl à 2 mM dans l'eau, [ascorbate finale]= 100 µM), sauf dans les témoins, avant de commencer la mesure de la fluorescence à l'aide de l'EasyQ (bioMérieux Cf exemple 1) pendant 120 minutes à 37°C. Ces mesures sont répétées quatre fois ainsi que les expériences témoin correspondantes ne contenant pas d'ascorbate. Seule la valeur moyenne est représentée sur les figures 5, 6 et 7.

**Tableau 2 : Mélanges d'oligonucléotides utilisés dans l'exemple 4.**

| | Mélange ODN | Molarité (mM) dans le tampon | Cliphen .Cu (µM) | Molarité Ascorbate (mM) | |
|---|---|---|---|---|---|
| ADN sonde fluorigène | 16025 | 10 | 1 | 0 | 100 |
| ARN sonde fluorigène | 16027 | 10 | 1 | 0 | 100 |
| ADN /ADN | 16025/16026 | 10 / 10 | 1 | 0 | 100 |
| ARN /ARN | 16027/16028 | 10 / 10 | 1 | 0 | 100 |
| ARN/ADN | 16027/16026 | 10 / 10 | 1 | 0 | 100 |

### Résultats et conclusions :

On observe sur la figure 5 le résultat de la mesure de fluorescence au cours du temps d'un simple brin ADN fluorigène (16025, courbe a) ou ARN fluorigène (16027, courbe b) soumis à l'action du mélange ClipPhen-Cu/ascorbate. On observe dans les deux cas une augmentation de fluorescence très nette en comparaison aux témoins (courbes c et d). Cette apparition de fluorescence démontre que l'ADN est fragmenté aussi bien que l'ARN et à une vitesse comparable pendant les premiers instants de la réaction.

La figure 6 montre la fragmentation d'un duplex ADN/ADN. Lors de la formation du duplex ADN/ADN (courbes a et b) nous observons que le niveau de fluorescence est augmenté d'un facteur 6 par rapport au témoin simple brin (courbes c ou d). Cette augmentation n'est pas due à la ClipPhen-Cu mais à l'hybridation entre les 2 brins. Dès l'ajoût d'ascorbate, on observe une diminution de fluorescence de la courbe b qui rejoint le niveau de fluorescence de la courbe d correspondant à la fluorescence émise par le simple brin totalement clivé. La modification de la fluorescence par rapport aux témoins démontre bien que le simple brin ADN et le double brin ADN/ADN sont clivés de façon absolument comparable.

Nous avons enfin évalué la différence de vitesse de fragmentation selon que les acides nucléiques à fragmenter sont de l'ADN ou de l'ARN double brin ou un hétéroduplex ARN/ADN (Figure 7). On observe dans ce cas, pour les 3 témoins et comme dans la Figure 6, une augmentation de fluorescence dès la formation des duplex puis, lors de la fragmentation induite par l'ajoût d'ascorbate, une diminution de fluorescence rejoignant le niveau de fluorescence d'un simple brin totalement clivé.
Dans tous les cas et quelle que soit la nature de l'hybride, la fragmentation a lieu, montrant que le mélange ClipPhen-Cu /Ascorbate est parfaitement adapté à la dégradation de n'importe quel type d'acides nucléiques.

### Exemple 5 Effet décontaminant d'une molécule de fragmentation immobilisée sur un support solide

**But :** Immobiliser la ClipPhen sur un support solide et démontrer le maintien de son activité après l'immobilisation sur billes de polystyrène Tentagel (référence commerciale MB 250 002, Rapp Polymers, Germany)

### Mode opératoire :

Placer 61,5mg de billes TentaGel Macrobeads NH₂ (référence commerciale MB 250 002 Rapp Polymers, Germany) dans une colonne Snap Fit (ABI, USA) ou une colonne Handee Centrifuge (référence 69705 Pierce, USA) soit n=16,6µmol. Laver les billes avec du diméthyl sulfoxyde (DMSO) anhydre afin d'enlever toute trace éventuelle d'eau. Soutirer le DMSO. Passer sous un flux d'argon. Percoler 14µL de triéthylamine (6eq, 99 µmol, ≈150mM) dans 600µL de DMSO anhydre (1min). Cette étape permet de s'assurer que les groupements amines sont bien sous la forme NH₂ et non NH₃⁺. Soutirer la solution de triéthylamine. Percoler 36,4mg de disuccinimidyl suberate (DSS) (6eq, 99 µmol, ≈250mM, Pierce) dans 400µL de DMSO anhydre (10 min). Soutirer la solution contenant le DSS en excès.

Un test à la ninhydrine sur quelques billes prélevées à ce stade de la réaction permet de s'assurer que l'ensemble des fonctions amines ont réagi avec le linker. Si ce n'est pas le cas, les billes se colorent en bleu.

Percoler pendant 1h minimum (1h15-1h30) à température ambiante un mélange réactionnel contenant : la 3-ClipPhen (2,8eq, 45µmol, 56 mM), 4-Dimethylaminopyridine (2,8eq, 45µmol, 56mM), 6,3µL de triéthylamine (2,8eq, 45µmol, 56mM) dans 800µL de DMSO.

Il est possible de prélever quelques µL de mélange réactionnel avant et après réaction afin de déterminer le taux de fonctionnalisation des billes par dosage différentiel en UV à 328nm. Le mélange réactionnel a une coloration brune due à la 3-ClipPhen. Laver au DMSO anhydre jusqu'à disparition de cette coloration brune de la solution de lavage.

Les billes initialement jaunâtres présentent une couleur jaune légèrement plus foncée après réaction. Ajouter 5,9 µL d'éthanolamine (10 eq, 99 µmol ≈ 130mM) dans 800µL de DMSO anhydre, afin d'inactiver tous les groupements NHS n'ayant pas réagi. Laisser agir 10 minutes. Laver les billes 5 fois avec 1mL de DMSO, soutirer le DMSO. Refaire la même opération avec de l'acétonitrile (afin de pouvoir sécher les billes). Passer sous un flux d'argon puis évaporer avec un évaporateur rotatif.

### Complexation au cuivre.

Percoler pendant 10 minutes 1mL d'une solution de CuCl₂ 1M dans DMSO/eau 50/50 (Sur 55mg de billes, soit 145 équivalents par rapport au taux de fonctionnalisation). Laver au DMSO et à l'eau jusqu'à ce que la solution de lavage soit incolore (si la solution CuCl₂ initiale était colorée) puis à l'acétonitrile. Passer sous un flux d'Argon. Sécher avec un évaporateur rotatif.

### Test de clivage.

Le test de clivage est réalisé comme suit : la quantité souhaitée de résine, entre 1mg et une seule bille soit une quantité de ClipPhen introduite de 125nmol/12,5mM pour la TentaGel, (soit une quantité de ClipPhen introduite de 1,25 à 2,5nmol/125µM à 250µM) est introduite dans un tube Eppendorf (contenance maximale 200µL). La cible ARN (transcrits ARN 16s de 1600 bases, concentration entre 6 et 70ng/µL selon les expériences) est ajoutée sur la résine, de même que l'ascorbate à la concentration souhaitée (5mM). Les échantillons ont un volume total de 10µL, le tampon utilisé est un tampon tris à pH=7,4, de concentration finale 20mM.

Une seule bille de résine TentaGel suffit à l'observation du clivage. Des échantillons témoins « cible seule » (electrophoregramme A figure 8), « cible en présence d'ascorbate » (electrophoregramme B), « cible en présence de résine » couplée au complexe ClipPhen-Cu sont réalisés. Le témoin « cible en présence d'ascorbate » permet de contrôler que la quantité d'ascorbate utilisée n'a pas d'effet sur la fluorescence. Les échantillons ainsi préparés sont mis à agiter 30 minutes à température ambiante (1000 rpm) avant d'être déposé sur puce Agilent RNA 6000 Nano ((Kit commercial référencé 5067-1512, Agilent, USA) selon la cible utilisée. 1µL de surnageant suffit à l'analyse sur Bioanalyseur (Agilent, USA).

### Résultats et conclusions :

Les résultats obtenus et représentés sur la figure 8 (Electrophoregramme C) montrent qu'il y a bien clivage de la cible considérée en une demi heure, uniquement en présence d'ascorbate, pour une quantité de résines minimales (1 seule bille pour la TentaGel, soit en considérant qu'1mg de résine contient entre 50 et 100 billes, une quantité de ClipPhen supportée comprise entre 1,25 et 2,5 nmol, soit une concentration comprise entre 125 et 250µM) Ce clivage est très facilement observable sur le modèle ARN, puisque les fragments générés sont visibles sur l'électrophoregramme (Electrophoregramme C).

On démontre ainsi que la ClipPhen immobilisée est encore active et a conservée ses propriétés de dégradation des acides nucléiques.

### Exemple 6 Démonstration de la décontamination d'une solution contenant des acides nucléiques contaminants et des acides nucléiques d'intérêt

**But :** Eliminer les contaminants « ADN cellulaire génomique » se trouvant dans le surnageant d'une culture virale sans dégrader des acides nucléiques viraux présents en plus petite quantité et protégés par une capside virale. Ceci dans le but de diminuer les amplifications non spécifiques qui se font à partir de l'ADN cellulaire sans inhiber l'amplification spécifique de la cible d'intérêt.

Pour cela on dose avant et après action du mélange ClipPhen-Cu/ascorbate la concentration d'ADN 18S génomique dans le surnageant (kit commercial de dosage Search LC + ADN génomique, Promega, réf G3041, USA) pour la réalisation d'une gamme de dilution.On dose en parallèle la concentration en ARN viral avant et après action du mélange ClipPhen-Cu/ascorbate, par PCR avec des primers spécifiques et un dosage au Light Cycler . SybrGreen (Roche, USA)

### Mode opératoire :

Des cellules infectées par un virus sont mises en culture. Dans une expérience témoin on dose dans le surnageant l'ADN génomique résiduel (18S) avec le Kit Search LC (Entrée B du tableau ci-dessous). On mesure également au Light Cycler (Roche) la quantité d'acides nucléiques correspondant au virus (technique d'amplification avec des primers spécifiques) (Entrée B du tableau ci-dessous).

Dans une autre expérience (Entrée A du tableau ci-dessous) on soumet le surnageant à l'action du mélange ClipPhen-Cu/ascorbate (ClipPhen-Cu 100µM, Ascorbate 50 mM, 30 min, inactivation au DTT avec 2,5M final) pendant 30 minutes, puis on mesure la quantité d'ADN génomique résiduel (18S) et la quantité d'acides nucléiques correspondant au virus.

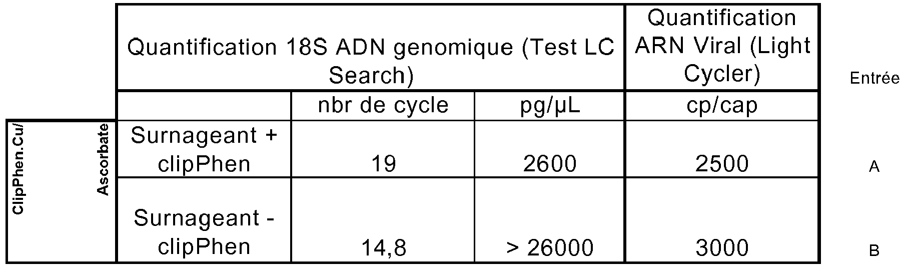

### Résultats et conclusions :

On constate que dans le cas où le surnageant a été traité avec l'agent de clivage la concentration en ADN génomique est 10 fois plus faible (Entrée A) sans que la concentration en acide nucléique viral ne soit affectée de façon conséquente.

Cette technique permet donc d'augmenter la sensibilité du dosage des acides nucléiques viraux en réduisant le bruit de fond de façon notable.

## Revendications

1. Méthode de décontamination d'une solution de tout acide nucléique présent dans cette solution comprenant les étapes suivantes :
- soumettre, pendant une durée suffisante, la solution à l'action d'au moins une molécule de fragmentation, formée par un complexe constitué de deux molécules de 1,10-phénanthroline associé à un atome métallique formant le complexe bis (1,10-phénanthroline)/métal, en présence de réducteur organique, jusqu'à la dégradation totale desdits acides nucléiques, et
- arrêter l'activité de la molécule de fragmentation par un ajout :
• d'un excès de réducteur organique, ou
• d'un agent complexant, tel que l'EDTA.

2. Méthode de décontamination d'une solution, contenant des acides nucléiques contaminants et des acides nucléiques d'intérêt comprenant les étapes suivantes :
- soumettre, pendant une durée suffisante, la solution à l'action d'au moins une molécule de fragmentation formée par un complexe constitué de deux molécules de 1,10-phénanthroline associé à un atome métallique formant le complexe bis (1,10-phénanthroline)/métal, en présence de réducteur organique, et ce jusqu'à la dégradation desdits acides nucléiques contaminants tout en conservant les acides nucléiques d'intérêt qui sont naturellement protégés de l'action de la molécule de fragmentation par une enveloppe extérieure, et
- arrêter l'activité de la molécule de fragmentation par un ajout :
• d'un excès de réducteur organique, ou
• d'un agent complexant, tel que l'EDTA.

3. Méthode, selon l'une quelconque des revendications 1 ou 2, **caractérisée en ce que** la méthode comprend, après l'arrêt de l'activité de la molécule de fragmentation, une étape supplémentaire qui consiste en une réaction d'amplification.

4. Méthode, selon l'une quelconque des revendications 1 à 3, **caractérisée en ce que** le métal est un métal de transition tel que le cuivre, le ruthénium, le nickel, le fer, le zinc, le rhodium, le cobalt, le manganèse.

5. Méthode, selon l'une quelconque des revendications 1 à 4, **caractérisée en ce que** les deux noyaux phénanthroline de la molécule de fragmentation sont reliés l'un à l'autre par un bras de liaison pour former une molécule ClipPhen.

6. Méthode, selon la revendication 5, **caractérisée en ce que** le bras de liaison entre les deux noyaux phénanthroline est constitué par une chaîne de trois atomes de carbone successifs, dont l'atome de carbone en position centrale est substitué et dont chaque atome de carbone terminal est relié à un noyau phénanthroline par un atome d'oxygène.

7. Méthode, selon la revendication 6, **caractérisée en ce que** l'atome de carbone en position centrale est substitué par -NH₂ ou -NH-CO-CH₃, et que chaque atome de carbone terminal est relié à un noyau phénanthroline par un atome d'oxygène en position 2 ou 3 dudit noyau.

8. Méthode, selon l'une quelconque des revendications 5 à 7, **caractérisée en ce que** la molécule ClipPhen est une 3-ClipPhen ((1,3-bis(1,10-phenanthrolin-3-yloxy)propan-2-amine).

9. Méthode, selon l'une quelconque des revendications 1 à 8, **caractérisée en ce que** la molécule de fragmentation est un complexe cuivreux de type I associé à du péroxyde d'hydrogène H₂O₂ et, éventuellement, à un autre réducteur, préférentiellement -organique.

10. Méthode, selon l'une quelconque des revendications 1 à 8, **caractérisée en ce que** la molécule de fragmentation est un complexe cuivrique de type II associé, préférentiellement, à un autre réducteur organique.

11. Méthode, selon l'une quelconque des revendications 1 à 10, **caractérisée en ce que** le réducteur tel que défini dans l'une quelconque des revendications 1 à 8, ou l'autre réducteur tel que défini dans l'une quelconque des revendications 9 ou 10, est un réducteur organique constitué par :
• un thiol, tel que le di-thiothréitol (DTT), un thio-acide, tel que l'acide mercapto-propionique ou
• un acide carboxylique ou
• un dérivé d'un acide carboxylique tel que l'ascorbate ou
• une phosphine comme la tri carboxy ethyl phosphine (TCEP), ou
• une combinaison d'au moins deux de ces réducteurs organiques.

12. Méthode, selon l'une quelconque des revendications 1 à 8, **caractérisée en ce que** la molécule de fragmentation est immobilisée sur un support solide.

13. Méthode, selon l'une quelconque des revendications 1 à 12, **caractérisée en ce que** le réducteur tel que défini dans l'une quelconque des revendications 1 à 8, ou l'autre réducteur tel que défini dans l'une quelconque des revendications 9 ou 10, est immobilisé sur un support solide.

14. Méthode, selon l'une quelconque des revendications 12 ou 13, **caractérisée en ce que** le support solide est une particule, une membrane, une bandelette, un film ou un filtre.

15. Méthode, selon l'une quelconque des revendications 9 ou 10, **caractérisée en ce que** le rapport entre la molécule de fragmentation et l'autre réducteur organique, constitué par un acide carboxylique ou un dérivé d'un tel acide, est compris entre 1 pour 1 et 1 pour 100.

16. Méthode, selon l'une quelconque des revendications 1 à 15, dans laquelle le rapport entre ladite molécule de fragmentation et le réducteur, selon l'une quelconque des revendications 1 à 8, constitué par un thiol, est supérieur à 1 pour 100, et préférentiellement supérieur à 1 pour 1000.

17. Méthode, selon l'une quelconque des revendications 1 à 16, **caractérisée en ce que** les acides nucléiques traités sont de l'ARN ou de l'ADN, se présentant sous forme simple ou double brin, ainsi que les hétéroduplexes ARN/ADN.

18. Méthode, selon l'une quelconque des revendications 1 à 16, **caractérisée en ce que** la durée du traitement subie par les acides nucléiques traités est comprise entre 5 et 60 minutes préférentiellement entre 10 et 30 minutes pour des concentrations en ClipPhen/métal comprises entre 1 µM et 100 µM.

19. Utilisation d'une solution traitée, par une méthode selon l'une quelconque des revendications 1 et 3 à 18, pour l'amplification d'acides nucléiques cibles, **caractérisée en ce que** la solution est mélangée avec un échantillon biologique contenant lesdits acides nucléiques cibles, que l'on souhaite amplifier, mais également les amorces d'amplification et les sondes de détection spécifiques des acides nucléiques cibles.

20. Utilisation, selon la revendication 19, **caractérisée en ce que** le réducteur organique, tel que décrit dans la méthode selon l'une quelconque des revendications 1 à 8, est identique à l'autre réducteur organique, selon l'une quelconque des revendications 9 ou 10.

21. Utilisation, selon la revendication 19, **caractérisée en ce que** le réducteur organique, tel que décrit dans la méthode selon l'une quelconque des revendications 1 à 8, est différent de l'autre réducteur organique, tel que décrit dans la méthode selon l'une quelconque des revendications 9 ou 10.

## Patentansprüche

1. Verfahren zur Dekontamination einer Lösung bezüglich jeglicher darin vorliegender Nukleinsäure, bei dem man:
- die Lösung über einen ausreichenden Zeitraum der Einwirkung mindestens eines Fragmentierungsmoleküls, gebildet durch einen Komplex aus zwei Molekülen 1,10-Phenanthrolin in Kombination mit einem Metallatom, das den Bis(1,10-phenanthrolin)/Metall-Komplex bildet, in Gegenwart eines organischen Reduktionsmittels bis zum vollständigen Abbau der Nukleinsäuren unterwirft und
- die Aktivität des Fragmentierungsmoleküls durch Zugabe
• eines Überschusses von organischem Reduktionsmittel oder
• eines Komplexbildners, wie EDTA, stoppt.

2. Verfahren zur Dekontamination einer Lösung, die kontaminierende Nukleinsäuren und Nukleinsäuren von Interesse enthält, bei dem man:
- die Lösung über einen ausreichenden Zeitraum der Einwirkung mindestens eines Fragmentierungsmoleküls, gebildet durch einen Komplex aus zwei Molekülen 1,10-Phenanthrolin in Kombination mit einem Metallatom, das den Bis(1,10-phenanthrolin)/Metall-Komplex bildet, in Gegenwart eines organischen Reduktionsmittels bis zum vollständigen Abbau der kontaminierenden Nukleinsäuren unter Konservierung der Nukleinsäuren von Interesse, die durch eine Außenhülle natürlich vor der Einwirkung des Fragmentierungsmoleküls geschützt sind, unterwirft und
- die Aktivität des Fragmentierungsmoleküls durch Zugabe
• eines Überschusses von organischem Reduktionsmittel oder
• eines Komplexbildners, wie EDTA, stoppt.

3. Verfahren nach einem der Ansprüche 1 oder 2, **dadurch gekennzeichnet, dass** das Verfahren nach dem Stoppen der Aktivität des Fragmentierungsmoleküls einen zusätzlichen Schritt umfasst, der aus einer Amplifikationsreaktion besteht.

4. Verfahren nach einem der Ansprüche 1 bis 3, **dadurch gekennzeichnet, dass** es sich bei dem Metall um ein Übergangsmetall wie Kupfer, Ruthenium, Nickel, Eisen, Zink, Rhodium, Cobalt oder Mangan handelt.

5. Verfahren nach einem der Ansprüche 1 bis 4, **dadurch gekennzeichnet, dass** die beiden Phenanthrolinkerne des Fragmentierungsmoleküls unter Bildung eines ClipPhen-Moleküls über einen Verknüpfungsarm miteinander verbunden sind.

6. Verfahren nach Anspruch 5, **dadurch gekennzeichnet, dass** der Verknüpfungsarm zwischen den beiden Phenanthrolinkernen aus einer Kette von drei aufeinanderfolgenden Kohlenstoffatomen besteht, wobei das Kohlenstoffatom in der zentralen Position substituiert ist und jedes endständige Kohlenstoffatom über ein Sauerstoffatom an einen Phenanthrolinkern gebunden ist.

7. Verfahren nach Anspruch 6, **dadurch gekennzeichnet, dass** das Kohlenstoffatom in der zentralen Position durch -NH₂ oder -NH-CO-CH₃ substituiert ist und jedes endständige Kohlenstoffatom über ein Sauerstoffatom an einen Phenanthrolinkern gebunden ist, wobei sich das Sauerstoffatom in Position 2 oder 3 des Kerns befindet.

8. Verfahren nach einem der Ansprüche 5 bis 7, **dadurch gekennzeichnet, dass** es sich bei dem ClipPhen-Molekül um ein 3-ClipPhen (1,3-Bis(1,10-phenanthrolin-3-yloxy)propan-2-amin) handelt.

9. Verfahren nach einem der Ansprüche 1 bis 8, **dadurch gekennzeichnet, dass** es sich bei dem Fragmentierungsmolekül um einen Kupfer(I)-Komplex vom Typ I in Kombination mit Wasserstoffperoxid H₂O₂ und gegebenenfalls einem anderen, vorzugsweise organischen, Reduktionsmittel handelt.

10. Verfahren nach einem der Ansprüche 1 bis 8, **dadurch gekennzeichnet, dass** es sich bei dem Fragmentierungsmolekül um einen Kupfer(II)-Komplex vom Typ II, vorzugsweise in Kombination mit einem anderen organischen Reduktionsmittel, handelt.

11. Verfahren nach einem der Ansprüche 1 bis 10, **dadurch gekennzeichnet, dass** es sich bei dem Reduktionsmittel, wie es in einem der Ansprüche 1 bis 8 definiert ist, bzw. dem anderen Reduktionsmittel, wie es in einem der Ansprüche 9 oder 10 ist, um ein organisches Reduktionsmittel, bestehend aus:
• einem Thiol, wie Dithiothreitol (DTT), einer Thiosäure, wie Mercaptopropionsäure, oder
• einer Carbonsäure oder
• einem Derivat einer Carbonsäure wie Ascorbat oder
• einem Phosphin wie Tricarboxyethylphosphin (TCEP) oder
• einer Kombination von mindestens zwei dieser organischen Reduktionsmittel,
handelt.

12. Verfahren nach einem der Ansprüche 1 bis 8, **dadurch gekennzeichnet, dass** das Fragmentierungsmolekül auf einem festen Träger immobilisiert ist.

13. Verfahren nach einem der Ansprüche 1 bis 12, **dadurch gekennzeichnet, dass** das Reduktionsmittel, wie es in einem der Ansprüche 1 bis 8 definiert ist, bzw. das andere Reduktionsmittel, wie es in einem der Ansprüche 9 oder 10 definiert ist, auf einem festen Träger immobilisiert ist.

14. Verfahren nach einem der Ansprüche 12 oder 13, **dadurch gekennzeichnet, dass** es sich bei dem festen Träger um ein Teilchen, eine Membran, einen Streifen, einen Film oder ein Filter handelt.

15. Verfahren nach einem der Ansprüche 9 oder 10, **dadurch gekennzeichnet, dass** das Verhältnis zwischen dem Fragmentierungsmolekül und dem anderen organischen Reduktionsmittel, das aus einer Carbonsäure oder einem Derivat einer derartigen Säure besteht, zwischen 1 zu 1 und 1 zu 100 liegt.

16. Verfahren nach einem der Ansprüche 1 bis 15, bei dem das Verhältnis zwischen dem Fragmentierungsmolekül und dem Reduktionsmittel nach einem der Ansprüche 1 bis 8, das aus einem Thiol besteht, größer als 1 zu 100 und vorzugsweise größer als 1 zu 1000 ist.

17. Verfahren nach einem der Ansprüche 1 bis 16, **dadurch gekennzeichnet, dass** es sich bei den behandelten Nukleinsäuren um RNA oder DNA in Einzel- oder Doppelstrangform sowie RNA/DNA-Heteroduplexe handelt.

18. Verfahren nach einem der Ansprüche 1 bis 16, **dadurch gekennzeichnet, dass** die Dauer der von den behandelten Nukleinsäuren durchlaufenen Behandlung für ClipPhen/Metall-Konzentrationen zwischen 1 µM und 100 µM zwischen 5 und 60 Minuten und vorzugsweise zwischen 10 und 30 Minuten liegt.

19. Verwendung einer durch ein Verfahren nach einem der Ansprüche 1 und 3 bis 18 behandelten Lösung zur Amplifikation von Zielnukleinsäuren, **dadurch gekennzeichnet, dass** man die Lösung mit einer biologischen Probe, die die zu amplifizierenden Zielnukleinsäuren, aber auch Amplifikationsprimer und für die Zielnukleinsäuren spezifische Detektionssonden enthält, mischt.

20. Verwendung nach Anspruch 19, **dadurch gekennzeichnet, dass** das organische Reduktionsmittel, wie es in dem Verfahren nach einem der Ansprüche 1 bis 8 beschrieben ist, mit dem anderen organischen Reduktionsmittel gemäß einem der Ansprüche 9 oder 10 identisch ist.

21. Verwendung nach Anspruch 19, **dadurch gekennzeichnet, dass** das organische Reduktionsmittel, wie es in dem Verfahren nach einem der Ansprüche 1 bis 8 beschrieben ist, von dem anderen organischen Reduktionsmittel gemäß einem der Ansprüche 9 oder 10 verschieden ist.

## Claims

1. Method for decontaminating a solution of any nucleic acid present in said solution, comprising the following steps:
- for a sufficient period, subjecting the solution to the action of at least one fragmentation molecule formed by a complex constituted by two molecules of 1,10-phenanthroline associated with a metal atom forming the bis(1,10-phenanthroline)/metal complex, in the presence of an organic reducing agent, until said nucleic acids have been completely degraded; and
- stopping the activity of the fragmentation molecule by adding:
• an excess of organic reducing agent; or
• a complexing agent such as EDTA.

2. Method for decontaminating a solution containing contaminating nucleic acids and nucleic acids of interest, comprising the following steps:
- for a sufficient period, subjecting the solution to the action of at least one fragmentation molecule formed by a complex constituted by two molecules of 1,10-phenanthroline associated with a metal atom forming the bis(1,10-phenanthroline)/metal complex, in the presence of an organic reducing agent, until said contaminating nucleic acids have been degraded, while conserving the nucleic acids of interest which are naturally protected from the action of the fragmentation molecule by an outer envelope; and
- stopping the activity of the fragmentation molecule by adding:
• an excess of organic reducing agent; or
• a complexing agent such as EDTA.

3. Method according to Claim 1 or Claim 2, **characterized in that** after stopping the activity of the fragmentation molecule, the method comprises a supplemental step which consists in an amplification reaction.

4. Method according to any one of Claims 1 to 3, **characterized in that** the metal is a transition metal such as copper, ruthenium, nickel, iron, zinc, rhodium, cobalt or manganese.

5. Method according to any one of Claims 1 to 4, **characterized in that** the two phenanthroline nuclei of the fragmentation molecule are connected to each other via a linking arm to form a ClipPhen molecule.

6. Method according to Claim 5, **characterized in that** the linking arm between the two phenanthroline nuclei is constituted by a chain of three successive carbon atoms wherein the carbon atom in the central position is substituted and wherein each terminal carbon atom is connected to a phenanthroline nucleus via an oxygen atom.

7. Method according to Claim 6, **characterized in that** the carbon atom in the central position is substituted with -NH₂ or -NH-CO-CH₃ and **in that** each terminal carbon atom is connected to a phenanthroline nucleus via an oxygen atom in position 2 or 3 of said nucleus.

8. Method according to any one of Claims 5 to 7, **characterized in that** the ClipPhen molecule is a 3-ClipPhen (1,3-bis(1,10-phenanthrolin-3-yloxy)propan-2-amine).

9. Method according to any one of Claims 1 to 8, **characterized in that** the fragmentation molecule is a type I copper complex associated with hydrogen peroxide H₂O₂ and, optionally, with another reducing agent, preferably organic.

10. Method according to any one of Claims 1 to 8, **characterized in that** the fragmentation molecule is a type II copper complex preferably associated with another organic reducing agent.

11. Method according to any one of Claims 1 to 10, **characterized in that** the reducing agent as defined in any one of Claims 1 to 8 or the other reducing agent as defined in Claim 9 or Claim 10 is an organic reducing agent constituted by:
• a thiol such as dithiothreitol (DTT), a thioacid such as mercaptopropionic acid; or
• a carboxylic acid; or
• a derivative of a carboxylic acid such as the ascorbate; or
• a phosphine such as tricarboxyethyl phosphine (TCEP); or
• a combination of at least two of said organic reducing agents.

12. Method according to any one of Claims 1 to 8, **characterized in that** the fragmentation molecule is immobilized on a solid support.

13. The method as claimed in any one of Claims 1 to 12, **characterized in that** the reducing agent as defined in any one of Claims 1 to 8 or the other reducing agent as defined in Claim 9 or Claim 10 is immobilized on a solid support.

14. Method according to Claim 12 or Claim 13, **characterized in that** the solid support is a particle, a membrane, a strip, a film or a filter.

15. The method according to Claim 9 or Claim 10, **characterized in that** the ratio between the fragmentation molecule and the other organic reducing agent, constituted by a carboxylic acid or a derivative of said acid, is in the range between 1 to 1 and 1 to 100.

16. The method according to any one of Claims 1 to 15, in which the ratio between said fragmentation molecule and the reducing agent according to any one of Claims 1 to 8, constituted by a thiol, is more than 1 to 100 and preferably more than 1 to 1000.

17. Method according to any one of Claims 1 to 16, **characterized in that** the nucleic acids which are treated are RNA or DNA in the single or double strand form, as well as RNA/DNA heteroduplexes.

18. Method according to any one of Claims 1 to 16, **characterized in that** the period for the treatment undergone by the treated nucleic acids is in the range 5 to 60 minutes, preferably in the range 10 to 30 minutes, for concentrations of ClipPhen/metal in the range 1 µM to 100 µM.

19. Use of a solution treated using a method according to any one of Claims 1 and 3 to 18, for the amplification of target nucleic acids, **characterized in that** the solution is mixed with a biological sample containing said target nucleic acids which it is desired to amplify, but also amplification primers and detection probes which are specific for the target nucleic acids.

20. Use according to Claim 19, **characterized in that** the organic reducing agent as described in the method according to any one of Claims 1 to 8 is identical to the other organic reducing agent according to Claim 9 or Claim 10.

21. Use according to Claim 19, **characterized in that** the organic reducing agent as described in the method according to any one of Claims 1 to 8 is different from the other organic reducing agent, as described in the method according to Claim 9 or Claim 10.


### SEQUENCE LISTING

<110> bioMérieux
<120> Méthode de décontamination d'une solution des acides nucléiques indésirables
<130> ClipPhen
<150> FR0754165
   <151> 2007-03-30
<160> 4
<170> PatentIn version 3.3
<210> 1
   <211> 27
   <212> DNA
   <213> Artificial
<220>
   <223> sonde
<400> 1
   tgtaatgatg agggtgtcac tgcggtt 27
<210> 2
   <211> 27
   <212> RNA
   <213> Artificial
<220>
   <223> sonde
<400> 2
   uguaaugaug agggugucac ugcgguu 27
<210> 3
   <211> 27
   <212> DNA
   <213> Artificial
<220>
   <223> cible
<400> 3
   aaccgcagtg acaccctcat cattaca 27
<210> 4
   <211> 27
   <212> RNA
   <213> Artificial
<220>
   <223> cible
<400> 4
   aaccgcagug acacccucau cauuaca 27